# EUROPÄISCHE PATENTSCHRIFT

(11) **EP 1 279 414 B1**
(45) Veröffentlichungstag und Bekanntmachung des Hinweises auf die Patenterteilung: **20.09.2006**
(21) Anmeldenummer: 02016293.9
(22) Anmeldetag: 23.07.2002
(51) Int. Cl.: A61N 1/39

(54) **Herztherapiegerät**
Cardiac therapy apparatus
Appareil de thérapie cardiaque

(30) Priorität: 25.07.2001 US 912056
(43) Veröffentlichungstag der Anmeldung: 29.01.2003
(73) Patentinhaber: BIOTRONIK GmbH & Co. KG, 12359 Berlin (DE)
(72) Erfinder: Shekhar, Mrigank, Vancouver, WA 98662 (US); Nigam, Indra B., Tigard, OR 97223 (US); Thong, Tran Dr., Portland, OR 97229 (US)
(74) Vertreter: Lindner-Vogt, Karin L.

(56) Entgegenhaltungen:
- EP-A- 0 360 412
- EP-A- 0 836 866
- EP-A- 0 919 256
- WO-A-99/47206
- US-A- 5 350 406
- US-A- 5 462 060
- US-A- 5 591 215
- US-A- 5 978 707
- US-B1- 6 230 055

## Beschreibung

Die Erfindung betrifft ein Herztherapiegerät nach dem Oberbegriff des Anspruchs 1, welches in der Praxis insbesondere als automatischer implantierbarer Kardioverter/Defibrillator (AICD) oder kombinierter Bedarfsschrittmacher/AICD ausgeführt ist.

Selbsttätig arbeitende Herztherapiegeräte sind - insbesondere und seit längerem als implantierbare Herzschrittmacher zur Behandlung von bradykarden und/oder tachykarden Herzrhythmusstörungen, aber auch zunehmend als automatische Defibrillatoren bzw. Kardioverter, als kombinierte Schrittmacher/Kardioverter oder als implantierte Medikamentendosierpumpen o. ä. - allgemein bekannt und im alltäglichen medizinischen Einsatz.

Bekannt sind insbesondere auch gattungsgemäße Geräte, die mit einem oder mehreren Fühler(n) zur Aufnahme des Herzrhythmus im Körper des Patienten und zugehörigen Signalaufbereitungs- und - verarbeitungseinrichtungen sowie einer Auswertungs- und Steuereinheit ausgerüstet sind. Diese berechnet gemäß einem im Gerät gespeicherten Algorithmus in Abhängigkeit vom Wert bzw. den Werten der Herzrate aus der Menge der programmierten Betriebsparameter bzw. Therapiegrößen jeweils einen aktuellen Parameter bzw. Parametersatz. Es sind Therapiegeräte dieser Art bekannt, die zu einer automatischen Aktivierung oder - insbesondere ebenfalls vorprogrammierten - Umschaltung aus einer Betriebsart in eine andere in Abhängigkeit vom im Körper des Patienten erfaßten Herzrhythmus ausgebildet. Hierzu zählen die bekannten Bedarfs-Herzschrittmacher oder automatischen Defibrillatoren und die in neuerer Zeit entwickelten Kombinationsgeräte.

Bereits seit der Entwicklung und dem klinischen Einsatz sogenannter Bedarfs-(Demand-)Schrittmacher ist es bekannt, speziell Herzschrittmacher derart zu steuern, daß die spontanen Herzaktionen erfaßt und daraus der Wert der Herzrate bzw. der Zeitintervalle zwischen bestimmten Herzaktionen (etwa der RR-Intervalle zwischen aufeinanderfolgenden Ventrikelaktionen) ermittelt und mit einem vorgegebenen Sollwert verglichen werden und der Schrittmacher genau dann Stimulationsimpulse abgibt, wenn der Meßwert nicht innerhalb eines durch den Sollwert begrenzten Bereiches liegt.

Modernere Geräte dieser Art sind mikroprozessorgesteuert und bieten die Möglichkeit oder individuellen, auf ein konkretes Krankheitsbild zugeschnittenen Programmierung einer von einer Mehrzahl vorinstallierter Betriebsweisen, mit der eine vorgegebene Therapie realisiert wird, und zugehöriger Betriebsparameter (im weiteren auch als Therapiegrößen bezeichnet, soweit sie therapeutisch relevant sind).

In diesem Rahmen haben auch wesentliche Weiterentwicklungen des Konzeptes des Bedarfs-Schrittmachers hin zum universellen Bedarfs-Herzrhythmuskorrekturgerät stattgefunden, von denen einige von einer fortschreitend feineren Bereichsunterteilung des Herzraten- oder RR-Intervall-Kontinuums ausgehen und in Abhängigkeit davon, in welchem der Mehrzahl vorgegebener Bereiche ein aktueller Meßwert liegt, eine von einer Mehrzahl definierter, jeweils eindeutig einem Bereich zugeordneter Therapien realisiert wird. Mit einem solchen Gerät ist ein klassischer Bedarfs-Schrittmacherbetrieb im Fall einer Bradykardie ebenso realisierbar wie konventionelle Therapien verschiedener Tachykardien (vgl. etwa US 4,181,133) oder gegebenenfalls auch eine Defibrillationsschock-Therapie (vgl. US 4,300,567).

Ausgehend von der Erkenntnis, daß allein die Zuordnung der Herzrate zu einem der vorgegebenen Bereiche nicht immer zuverlässig die Bestimmung der angemessenen Therapie erlaubt, wurden in den Entwicklungen der letzten Jahre verstärkt auch zusätzliche Klassifizierungskriterien getestet und in den Steueralgorithmen berücksichtigt; vgl. hierzu etwa US 5,379,776 (einschließh darin genannter Quellen).

Geräte der genannten Art werden bei der Implantation entsprechend dem Krankheitsbild und gegebenenfalls den Lebensbedingungen (beispielsweise der durchschnittlichen körperlichen Aktivität) des Patienten programmiert, wobei auch der anzuwendende Algorithmus zur Bestimmung der Therapie bzw.Therapiegröße(n) in Abhängigkeit vom Wert der im Körper erfaßten Größe(n) festgelegt wird. Bei den in bestimmten Abstanden erfolgenden Nachsorgeuntersuchungen können durch eine Umprogrammierung sowohl der Betriebsart- und -parametersatz als auch - falls das Therapiegerät über mehrere gespeicherte Algorithmen verfügt - der anzuwendende Steueralgorithmus geändert werden.

Aus der US 5,354,316 sind ein Verfahren und eine Vorrichtung zur Erfassung und Behandlung von Tachykardien und Fibrillationen eines Herzens mittels spezifisch ausgewählter Therapien eines Schrittmacher-Kardioverter-Defibrillator-Kombinationsgerätes (PCD) der gattungsgemäßen Art bekannt. Hierbei wird mit einander überlappenden Bereichsgrenzen zwischen Tachykardieund Fibrillations-Herzratenbereichen gearbeitet. Die Beurteilung des aktuell erfaßten Herzrhythmus - im Falle hoher Herzraten - zum Bereich der ventrikulären Tachykardie oder der ventrikulären Fibrillation erfolgt in den Überlappungsbereichen anhand zusätzlicher Beurteilungskriterien. Speziell wird untersucht, wie viele der vorangehenden Intervalle zwischen aufeinanderfolgenden Kammeraktionen in den Überlappungsbereich fallen.

Aus der US 5,447,519 ist ein ähnliches Verfahren bekannt, wobei hier als Unterscheidungskriterium zwischen einer ventrikulären Tachykardie und Kammerflimmern (ventrikulärer Fibrillation) die Variabilität der Morphologie des Herzsignals (EKG-Kurvenverlaufs) herangezogen wird.

Eine weitere gattungsgemäße Vorrichtung wird in der DE 196 09 362 C1 der Anmelderin beschrieben. Dieses Herztherapiegerät definiert ebenfalls einen Überlappungsbereich zwischen zwei aneinander angrenzenden Wertebereichen einer im Körper eines Patienten gemessenen Größe. Es lehrt für die Steuerung einer ausgewählten, vorbestimmten Therapie dann, wenn die Größe in den Überlappungsbereich fällt, allgemein eine Auswertung der Vorgeschichte der Meßgröße (speziell der Bereichszuordnung des jeweils vorhergehenden Wertes) oder aber der Therapiesteuergröße selbst zugrunde.

Dokument US 5978707 offenbart ein System gemäß dem Oberbegriff des Anspruchs 1.

Der Erfindung liegt die Aufgabe zugrunde, ein verbessertes Therapiegerät der gattungsgemäßen Art anzugeben, welches einen relativ einfachen und kostengünstigen Aufbau hat und gleichwohl eine möglichst schonende, aber zugleich ... wirksame Therapie tachykarder Herzrhythmusstörungen im Grenzbereich zu Fibrillationen bei einer atrialen Tachykardie zu liefern vermag.

Diese Aufgabe wird durch ein Herztherapiegerät mit den Merkmalen des Anspruchs 1 gelöst.

Die Erfindung schließt den wesentlichen Gedanken ein, eine Auswertung hoher Herzraten zur Ableitung einer adäquaten Therapie unter Einteilung des entsprechenden (hochfrequenten) Herzratenbereiches in drei aneinander angrenzende Bereiche mit festen Bereichsgrenzen vorzunehmen. Sie kehrt sich damit von früheren Lösungen nach den oben angegebenen Druckschriften ab, bei denen Überlappungsbereiche mit insbesondere nicht starr vorgegebenen Grenzen definiert wurden. Weiterhin schließt die Erfindung den Gedanken ein, im mittleren der drei Wertebereiche zur Ableitung einer geeigneten Therapie neben der Herzrate selbst das Ergebnis einer statistischen Auswertung derselben - und zwar speziell deren Stabilität in einem vorhergehenden, festgelegten Zeitraum - heranzuziehen. Mit diesem Ansatz wird eine Vereinfachung der Hard- und Softwarekonfiguration des Herztherapiegerätes und eine Erhöhung der Vorhersagbarkeit und Zuverlässigkeit der Therapie erreicht.

Erfindungsgemäß ist vorgesehen, daß bei Erfassung einer im ersten Wertebereich (Tachykardiezone 1) liegenden Herzrate ein erstes, eindeutig vorbestimmtes Therapiesteuersignal und bei Erfassung im dritten Wertebereich (Fibrillationszone) liegenden Herzrate ein drittes, ebenfalls eindeutig bestimmtes Therapiesteuersignal ausgegeben wird. Wird aber eine im zweiten Wertebereich (Tachykardiebereich 2) liegende Herzrate erfaßt, wird eines von zwei verschiedenen Therapiesteuersignalen ausgegeben - und zwar in Abhängigkeit von der Stabilität der Herzrate. Das Stabilitätskriterium dient zur Unterscheidung zwischen "schneller" Tachykardie ("Flutter") und Fibrillationen mit - noch - relativ geringer Frequenz. Es ist insbesondere vorgesehen, im Falle hoher Stabilität ein zweites, spezielles Therapiesteuersignal zur Einleitung einer auf die schnelle Tachykardie zugeschnittenen Korrekturmaßnahme auszugeben, während im Falle geringer Stabilität ebenso das dritte Therapiesteuersignal ausgegeben wird wie bei einer im dritten Wertebereich liegenden Herzrate.

Dieses dritte Therapiesteuersignal wird in der Regel die Einleitung einer Schocktherapie bewirken, also eine Kardioverter- bzw. Defibrillatorstufe des Herztherapiegerätes aktivieren. Dem gegenüber wird mit dem ersten Therapiesteuersignal u. U. überhaupt noch keine Stimulation tatsächlich eingeleitet, sondern das Therapiegerät gegebenenfalls erst in einen Bereitschaftszustand versetzt. Es kann aber auch bereits mit dem ersten Therapiesteuersignal die Abgabe von Schrittmacherimpulsfolgen an das Herz gesteuert werden - beispielsweise zur frühzeitigen Bekämpfung einer sogenannten Re.entry-Tachykardie. Mit dem zweiten Therapiesteuersignal wird eine Therapie gesteuert, welche in ihrer "Aggressivität" zwischen den Therapien liegt, die in der Tachykardiezone 1 einerseits und der Fibrillationszone andererseits angewandt werden. Es kann sich dabei einerseits noch um Schrittmacherimpulsfolgen, beispielsweise um die (an sich bekannten) Hochfrequenz-Burst- oder Ramp-Folgen, handeln - andererseits aber auch um eine Schocktherapie (Kardioversion) mit einem oder mehreren Schockimpuls(en) relativ geringer Energie. (Auch das Konzept der niederenergetischen Kardioversion ist an sich bekannt und bedarf daher hier keiner weiteren Erläuterung.)

Die oben skizzierte Funktion des Therapiegerätes beruht also auf der Erkenntnis, daß es für den Vorhofrhythmus zwischen dem Bereich nicht lebensbedrohlicher ("physiologischer") Tachykardie und dem Bereich lebensbedrohlicher Fibrillationen einen Ratenbereich hochfrequenter Tachykardie gibt, in dem das Stabilitätskriterium zur Unterscheidung zwischen Tachykardie und Fibrillationen und zu einer entsprechenden Differenzierung der Therapie herangezogen wird. Ein stabiler atrialer Rhythmus wird als Vorhofflattern (atrial flutter) durch eine Schrittmacherstufe des Therapiegerätes behandelt, wodurch für diesen Zustand unnötige und für den Patienten schmerzhafte Kardioversionsschocks vermieden werden können. Vergleichbare Herzraten mit geringer Stabilität sind hingegen Indiz für ein bedrohliches Vorhofflimmern (atrial fibrillation) und erfordern die Aktivierung einer Kardioversions- bzw. Defibrillatorstufe.

Aus den vorstehenden Ausführungen ergibt sich bereits, daß in der Ausführung des Gerätes die Herzraten-Erfassungseinrichtung zur Erfassung der atrialen Rate mit dem Vorhof des Herzens des Patienten verbunden ist - und zwar in der praktisch bedeutsamsten Ausführung als implantierbares Gerät in der Regel über eine innerhalb des Vorhofes plazierte Erfassungselektrode und eine mit dieser verbundene Meßsignalleitung.

Eine wesentliche Komponenten des vorgeschlagenen Herztherapiegerätes zur Verwirklichung dieses Therapiekonzeptes ist ein Dreibereichsspeicher zur Speicherung des ersten, zweiten und dritten Wertebereiches von Vergleichs-Herzraten, die den Gesamt-Frequenzbereich von relativ niederfrequenter, physiologischer Tachykardie bis zu extrem hochfrequenten Fibrillationen abdecken. Die Bereichsgrenzen zwischen den Bereichen sind für den laufenden Betrieb fest - gegebenenfalls aber durch Programmierung einstellbar - vorgegeben, und zwar entsprechend der konkreten Indikation in Abstimmung auf die konkreten Gegebenheiten beim jeweiligen Patienten. Der Dreibereichsspeicher wirkt mit einem mehrstufigen Herzratendiskriminator zusammen, welcher die Zuordnung einer gemessenen Herzrate zu einem der Wertebereiche vornimmt und ein das Zuordnungsergebnis charakterisierendes Ausgangssignal liefert. Dieses Ausgangssignal ist insbesondere zugleich das erste bzw. dritte Therapiesteuersignal, sofern die aktuell erfaßte Herzrate im ersten oder dritten Wertebereich liegt.

Mit dem Herzratendiskriminator arbeitet desweiteren eine Stabilitäts-Auswertungseinrichtung zur Auswertung der Herzraten-Stabilität über einen vorbestimmten Zeitraum (oder eine vorbestimmte Anzahl von vorangehenden Herzaktionen) zusammen. Diese wird aktiviert, sofern ein entsprechendes Ausgangssignal des Herzratendiskriminators eine gemessene Herzrate im zweiten Wertebereich anzeigt. Die Stabilitäts-Auswertungseinrichtung liefert ein die Stabilität der Herzrate charakterisierendes Ausgangssignal, welches in einer logischen Verarbeitungseinheit zusammen mit dem Diskriminator-Ausgangssignal verarbeitet wird, um im Ergebnis ein sowohl den Herzratenwert als auch dessen Stabilität reflektierendes Therapiesteuersignal zu gewinnen. Mit der Stabilitäts-Auswertungseinrichtung und der logischen Verarbeitungseinheit wird also gewissermaßen ein "Therapie-Umschalter" zwischen einer weniger aggressiven und einer aggressiveren Herzrhythmus-Korrekturmaßnahme zwischen Vorhof-Flattern einerseits und Vorhof-Flimmern andererseits realisiert.

Die Stabilitäts-Auswertungseinrichtung umfaßt die zur statistischen Auswertung der in einem bestimmten Zeitraum erfaßten Herzratenwerte erforderlichen Funktionskomponenten, und zwar insbesondere einen Herzraten-Meßwertspeicher, eine mit diesem verbundene statistische Verarbeitungseinheit zur Bestimmung eines Stabilitätswertes und einer mit dieser verbundene Stabilitäts-Diskriminatorstufe, in der der bei der statistischen Auswertung erhaltene tatsächliche Stabilitätswert mit einem vorbestimmten (programmierten) Schwellwert verglichen wird.

Die Therapieeinrichtung des Gerätes umfaßt mindestens einen Defibrillator, in der bevorzugten Ausführung aber zudem eine Schrittmachereinrichtung sowie insbesondere auch einen Therapiespeicher, aus dem im Ansprechen an die Therapiesteuersignale jeweils eine vorbestimmte Therapie des Defibrillators oder wahlweise Schrittmachers aktiviert wird. Der Therapiespeicher ist mit dem Ausgang der Auswertungs- und Steuereinrichtung auf solche Weise verbunden, daß die Therapiesteuersignale jeweils einen vorbestimmten Speicherbereich adressieren, in dem Therapiedaten einer konkreten Therapie abgelegt sind. Die Adressierung des ersten und dritten Speicherbereiches erfolgt direkt von der Herzraten-Diskriminatorstufe aus, während zur Adressierung des zweiten Speicherbereiches notwendigerweise und zur Adressierung des dritten Speicherbereiches zusätzlich das Ausgangssignal der Stabilitäts-Auswertungseinrichtung herangezogen wird. Hier sind also entsprechende UND-Glieder vorgesehen.

Bezüglich der konkreten Therapien und damit auch der im Therapiespeicher abgelegten Therapiedaten bestehen im Rahmen der Erfindung breite Variationsmöglichkeiten, die vom konkreten Einsatz des Gerätes zur Therapie von Vorhof-Rhythmusstörungen und von der Ausführung als reiner Kardioverter/Defibrillator oder kombinierter Bedarfs-Schrittmacher/Defibrillator abhängen. So kann bei einem reinen Defibrillator das erste Therapiesteuersignal ein Versetzen in den Bereitschaftszustand bewirken, während das zweite und dritte Therapiesteuersignal Schocktherapien mit unterschiedlichen Impulsenergien auslösen. Bei einem kombinierten Schrittmacher/Defibrillator werden dagegen das erste und zweite Therapiesteuersignal bevorzugt eine erste und zweite Stimulationsimpulsfolge auslösen, während das dritte Therapiesteuersignal einen Schockimpuls auslöst. Im übrigen wird zu diesen Möglichkeiten auf die grundlegenden Ausführungen weiter oben verwiesen.

Eine zuverlässige Funktion des vorgeschlagenen Gerätes erfordert die permanente oder periodische Abfrage der Herzrate, wobei zumindest bei Feststellung einer in den Gesamt-Wertebereich erhöhter Herzraten fallenden Herzrate die erwähnte Wertebereichs-Diskriminierung und gegebenenfalls Stabilitätsauswertung ausgelöst werden. Hierzu ist eine geeignete Ablaufsteuerung (Controller) in der Auswertungs- und Steuereinrichtung vorhanden.

Vorteile und Zweckmäßigkeiten der Erfindung ergeben sich im übrigen aus den Unteransprüchen sowie der nachfolgenden Beschreibung eines bevorzugten Ausführungsbeispiels anhand der Figuren. Von diesen zeigen:
- Fig. 1: eine schematische Darstellung des in verschiedene Wertebereiche gegliederten Kontinuums von Vorhofintervallen bzw. Vorhofraten zur Illustration der Arbeitsweise des vorgeschlagenen Herztherapiegerätes,
- Fig. 2: eine schematische Darstellung eines Herztherapiegerätes gemäß einer Ausführungsform der Erfindung in Art eines Funktions-Blockschaltbildes

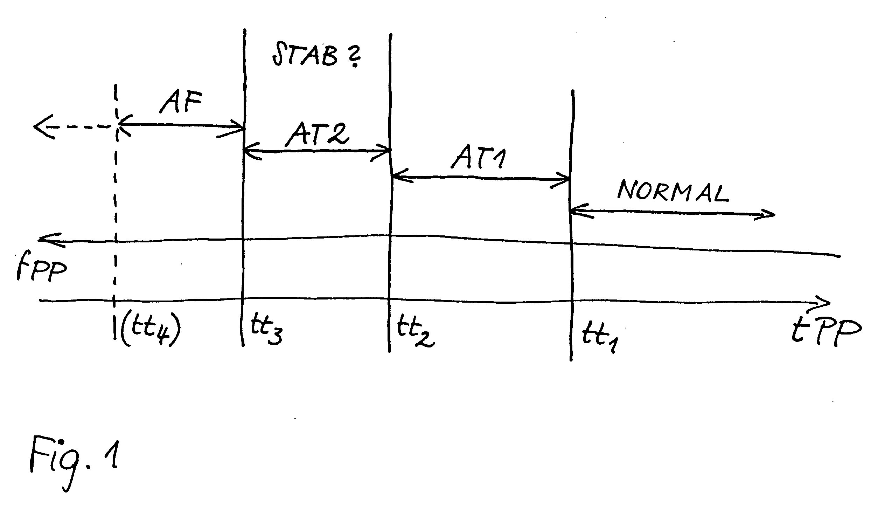

Fig. 1 zeigt schematisch, wie gemäß einer praktisch bedeutsamen Ausführung der Erfindung das Zeit- bzw. Frequenzkontinuum tPP bzw. fPP von Vorhofaktionen zur Einleitung geeigneter Therapien in aneinander angrenzende Wertebereiche gegliedert ist. Auf der x-Achse ist (mit nach rechts zunehmenden Werten) das PP-Intervall bzw. (mit nach links zunehmenden Werten) die atriale Herzrate fPP aufgetragen. Mit NORMAL ist der Bereich normaler Vorhofraten bezeichnet, mit AT1 und AT2 sind zwei aneinander angrenzende Bereiche atrialer Tachykardie mit unterschiedlicher diagnostischer und therapeutischer Relevanz bezeichnet, und AF bezeichnet den Bereich atrialer Fibrillation.

Die Grenzen tt₁ zwischen den Bereichen NORMAL und AT1, tt₂ zwischen den Bereichen AT1 und AT2 und tt₃ zwischen den Bereichen AT2 und AF sind erfindungsgemäß ohne Überlappungen eindeutig festgelegt. Für den Bereich AF kann aus verarbeitungstechnischen Gründen eine Obergrenze tt₄ festgelegt sein - was in der Figur durch eine gestrichelte Linie und die In-Klammern-Setzung von tt₄ symbolisiert ist -, dieser dritter Wertebereich kann aber auch nach oben offen sein. Zur Auswertung und in therapeutischen Maßnahmen in den einzelnen Bereichen wird auf die Erläuterungen weiter oben verwiesen.

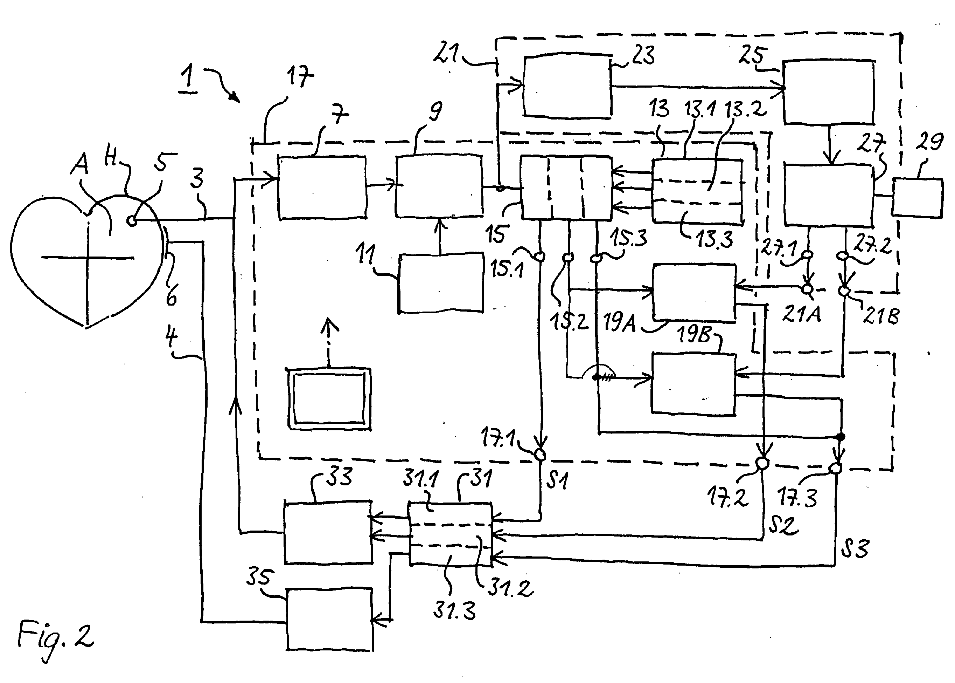

Fig. 2 zeigt in einer schematischen, lediglich als Prinzipskizze zu verstehenden Darstellung die für die Ausführung der Erfindung wesentlichen Funktionskomponenten eines implantierten kombinierten Vorhofschrittmachers/Defibrillators 1. Dieser ist ein- und ausgangsseitig über eine Elektrodenleitung 3, die sowohl als Meßsignal- wie auch als Stimulationsleitung wirkt, mit einer im Vorhof A eines Herzens H angeordneten Abfühl- und Stimulationselektrode 5 verbunden. Ausgangsseitig ist er zusätzlich über eine weitere Elektrode 4 mit einer Defibrillations- bzw. Schockelektrode 6 am Herzen H verbunden.

Die Elektrodenleitung 3 ist mit einer Eingangsstufe 7 verbunden, die (in an sich bekanntem Aufbau) Filter- und Verstärkerstufen zur Signalaufbereitung aufweist und an deren Ausgang ein störbefreites, pegelangepaßtes Vorhofsignal bereitsteht. Der Ausgang der Eingangsstufe 7 ist mit dem Eingang einer Zählerstufe 9 verbunden, deren Takteingang mit einem Takt- bzw. Zeitgeber 11 verbunden ist und in der die Rate fPP der erfaßten Vorhofaktionen (P-Zacken des Herzsignals) ermittelt wird.

Ein Vorhofraten-Vergleichswertspeicher 13 mit drei Speicherbereichen 13.1, 13.2 und 13.3 ist mit einem ersten Eingang einer Vorhofraten-Diskriminatorstufe 15 verbunden, und deren zweiter Eingang ist mit dem Ausgang der Zählerstufe 9 verbunden. Die Diskriminatorstufe 15 ist - was durch gestrichelte Linien innerhalb des Blockes 15 symbolisch dargestellt ist - mehrstufig aufgebaut und liefert ein Ausgangssignal, welches die Zugehörigkeit der aktuell gemessenen Vorhofrate zu einem der in den Speicherbereichen 13.1 bis 13.3 definierten Wertebereiche der Vorhofrate (vgl. Fig. 1) kennzeichnet. In der Figur ist dieser Umstand durch die Erstellung dreier verschiedener Ausgänge 15.1, 15.2 und 15.3 der Diskriminatorstufe 15 verdeutlicht.

Der Ausgang 15.1 der Diskriminatorstufe 15 bildet zugleich einen ersten Ausgang 17.1 der Auswertungs- und Steuereinrichtung 17 des Vorhofschrittmachers/Defibrillators 1, an der ein erstes Therapiesteuersignal S1 bereitgestellt wird. Der Ausgang 15.3 der Diskriminatorstufe 15 ist mit einem weiteren Ausgang 17.3 der Auswertungs- und Steuereinrichtung verbunden, an dem ein drittes Therapiesteuersignal S3 bereitgestellt wird. Der Ausgang 15.2 der Diskriminatorstufe 15 ist über ein erstes UND-Gatter 19A mit einem weiteren Ausgang 17.2 verbunden, an dem ein zweites Therapiesteuersignal S2 bereitgestellt wird. Schließlich verbindet ein weiteres UND-Gatter 19B den Ausgang 15.2 der Diskriminatorstufe mit dem bereits erwähnten Ausgang 17.3 der Auswertungs- und Steuereinrichtung.

Die UND-Gatter 19A, 19B sind jeweils an ihrem zweiten Eingang mit einem Ausgang 21A, 21B einer Stabilitäts-Auswertungseinrichtung 21 verbunden. Durch diese Anordnung wird die Ausgabe des erwähnten dritten Therapiesteuersignals S3 (neben dem direkten Weg) an eine logische Verknüpfung des Ausgangssignals der Diskriminatorstufe 15 und eines Ausgangssignals der Stabilitäts-Auswertungseinrichtung 21 gebunden, und ein zweites Therapiesteuersignal S2 wird notwendigerweise im Ergebnis einer logischen Verknüpfung der Ausgangssignale am zweiten Ausgang 17.2 der Diskriminatorstufe 15 sowie der Stabilitäts-Auswertungseinrichtung 21 geknüpft. (Zum Gegenstand und Hintergrund dieser logischen Verknüpfung wird auf die Ausführungen weiter oben verwiesen.)

Die Stabilitäts-Auswertungseinrichtung 21 umfaßt einen eingangsseitig mit der Zählerstufe 9 verbundenen Vorhofraten-Meßwertspeicher 23 und eine eingangsseitig mit diesem verbundene statistische Verarbeitungseinheit 25 zur Berechnung eines Stabilitätswertes der im Meßwertspeicher 23 abgelegten, in einem vorbestimmten Vergangenheits-Zeitraum erfaßten Vorhofratenwerte. Mit dem Ausgang der Verarbeitungseinheit 25 ist ein erster Eingang einer Stabilitäts-Diskriminatorstufe 27 verbunden, deren anderer Eingang mit einem Stabilitäts-Schwellwertspeicher 29 verbunden ist und die eine Schwellwertdiskriminierung des im Ergebnis der statistischen Verarbeitung in der Verarbeitungseinheit 25 erhaltenen Stabilitätswertes an einem vorprogrammierten Schwellwert ausführt.

Das Ergebnis der Auswertung bilden - in Abhängigkeit davon, ob der ermittelte Stabilitätswert unterhalb oder oberhalb des Schwellwertes liegt - zwei verschiedene Ausgangssignale, was in der Figur wiederum durch die Darstellung zweier Ausgänge 27A, 27B der Diskriminatorstufe 27 verdeutlicht ist. Der Ausgang 27A ist mit dem zweiten Eingang des UND-Gatters 19A verbunden, und der Ausgang 27B ist mit dem zweiten Eingang des UND-Gatters 19B verbunden. Im Falle hoher Stabilität der Vorhofrate wird das UND-Gatter 19A mit einem Ausgangssignal beaufschlagt und hierdurch das zweite Therapiesteuersignal S2 erzeugt, während im Falle niedriger Stabilität der Vorhofrate das UND-Gatter 19B mit einem Ausgangssignal der Stabilitäts-Diskriminatorstufe 27 beaufschlagt und das dritte Therapiesteuersignal S3 erzeugt wird.

Mit den Ausgängen der Auswertungs- und Steuereinrichtung 17 ist ein Therapiespeicher 31 mit drei Speicherbereichen 31.1, 31.2, 31.3 verbunden, in dessen Speicherbereichen die Therapiedaten für unterschiedliche Therapien zur Korrektur überhöhter Vorhofraten gespeichert sind. Konkret ist der Speicherbereich 31.1 eingangsseitig mit dem Ausgang 17.1 verbunden, der Speicherbereich 31.2 ist mit dem Ausgang 17.2 verbunden, und der Speicherbereich 31.3 ist mit dem Ausgang 17.3 verbunden. Die Speicherbereiche 31.1, 31.2 enthalten jeweils einen Therapiedatensatz für verschiedene Schrittmacher-Impulsfolgen, und der Speicherbereich 31.3 enthält einen Therapiedatensatz für eine Schockimpulstherapie. Die Daten aus den Speicherbereichen 31.1, 31.2 werden einer Schrittmacherstufe 33 des kombinierten Vorhofschrittmachers/Defibrillators 1 zugeführt, die daraufhin die jeweils spezifizierte Schrittmachertherapie über die Elektrodenleitung 3 und die Abfühl- und Stimulationselektrode 5 an den Vorhof A ausgibt. Die Therapiedaten aus dem Speicherbereich 31.3 werden einer Defibrillatorstufe 35 zugeführt, welche einen entsprechend den Daten konfigurierten Schockimpuls über die weitere Elektrodenleitung 4 und die spezielle Schockelektrode 6 an den Vorhof anlegt.

Eine Ablaufsteuereinheit 37 steuert die oben genannten Ratenerfassungs- und -verarbeitungsvorgänge, die Gewinnung der Therapiesteuersignale und die Ausgabe der entsprechenden Therapie an das Herz H des Patienten.

### Bezugszeichenliste

- 1: Vorhofschrittmacher/Defibrillator
- 3, 4: Elektrodenleitung
- 5: Abfühl- und Stimulationselektrode
- 6: Schockelektrode
- 7: Eingangsstufe
- 9: Zählerstufe
- 11: Takt- bzw. Zeitgeber
- 13: Vorhofraten-Vergleichswertspeicher
- 13.1, 13.2, 13.3: Speicherbereich
- 15: Vorhofraten-Diskriminatorstufe
- 15.1, 15.2, 15.3: Ausgang
- 17: Auswertungs- und Steuereinrichtung
- 17.1, 17.2, 17.3: Ausgang
- 19A, 19B: UND-Gatter
- 21: Stabilitäts-Auswertungseinrichtung
- 21A, 21B: Ausgang
- 23: Vorhofraten-Meßwertspeicher
- 25: statistische Verarbeitungseinheit
- 27: Stabilitäts-Diskriminatorstufe
- 27A, 27B: Ausgang
- 29: Stabilitäts-Schwellwertspeicher
- 31: Therapiespeicher
- 31.1, 31.2, 31.3: Speicherbereich
- 33: Schrittmacherstufe
- 35: Defibrillatorstufe
- 37: Ablaufsteuereinheit
- A: Vorhof
- AF: Fibrillationsbereich
- AT1: Tachykardiezone 1
- AT2: Tachykardiezone 2
- fPP: Vorhofrate
- H: Herz
- S1, S2, S3: Therapiesteuersignale
- tt₁, tt₂, tt₃, tt₄: Wertebereichsgrenze
- tPP: Vorhofintervall

## Patentansprüche

1. Herztherapiegerät mit
- einer Herzraten-Erfassungseinrichtung zur Erfassung der Herzrate eines Patienten,
- einer mindestens mittelbar mit dem Ausgang der Herzraten-Erfassungseinrichtung verbundenen Auswertungs- und Steuereinrichtung zur Auswertung einer gemessenen Herzrate, einschließlich deren Zuordnung zu einem von mindestens zwei vorbestimmten Wertebereichen, und zur Ausgabe einem Therapiesteuersignal in Abhängigkeit von dieser Zuordnung und
- einer mit dem Ausgang der Auswertungs- und Steuereinrichtung verbundenen Therapieeinrichtung zur Realisierung mindestens einer Therapie mit einer vorbestimmten Therapiegröße im Ansprechen auf die Therapiesteuergröße, also in Abhängigkeit von der Zugehörigkeit der gemessenen Herzrate zu einem der Wertbereiche,
wobei die Auswertungs- und Steuereinrichtung
- einen Dreibereichsspeicher zur Speicherung eines ersten, zweiten und dritten, mit aufsteigenden Werten der Herzrate aneinander angrenzenden Wertebereiches von Vergleichs-Herzraten,
- einen mit der Herzraten-Erfassungseinrichtung und dem Dreibereichsspeicher verbundenen Herzratendiskriminator zur Zuordnung einer gemessenen Herzrate zu dem ersten, zweiten oder dritten Wertebereich und zur Ausgabe eines entsprechenden ersten, zweiten oder dritten Diskriminator-Ausgangssignals,
- eine Stabilitäts-Auswertungseinrichtung zur Auswertung der Herzraten-Stabilität über einen vorbestimmten Zeitraum im Ansprechen auf die Ausgabe des zweiten Herzratendiskriminator-Ausgangssignals und
- eine logische Verarbeitungseinheit zur Verarbeitung des zweiten Diskriminator-Ausgangssignals und eines Ausgangssignals der Stabilitäts-Erfassungseinrichtung zur Erzeugung eines zweiten oder dritten Therapiesteuersignal, während das erste und dritte Diskriminator-Ausgangssignal direkt als erstes und drittes Therapiesteuersignal ausgegeben werden,
aufweist, **dadurch gekennzeichnet, daß** die Herzraten-Erfassungseinrichtung, ausgebildet ist, insbesondere über mindestens eine Erfassungselektrode und eine mit dieser verbundene Meßsignalleitung, zur Erfassung der atrialen Herzrate mit dem Vorhof des Herzens des Patienten verbunden zu sein und daß die Therapieeinrichtung zur Ausgabe mindestens zweier verschiedener, eindeutig vorbestimmter Therapien im Ansprechen auf das zweite und dritte Therapiesteuersignal ausgebildet ist.

2. Herztherapiegerät nach Anspruch 1,
**dadurch gekennzeichnet, daß**
die Stabilitäts-Auswertungseinrichtung
- einen Herzraten-Meßwertspeicher zur Speicherung einer vorbestimmten Anzahl von Herzraten-Meßwerten oder aller Herzraten-Meßwerte in einem vorbestimmten Zeitraum,
- eine mit dem Herzraten-Meßwertspeicher verbundene statistische Verarbeitungseinheit zur statistischen Verarbeitung und Bestimmung eines Stabilitätswertes der Herzrate und
- eine Stabilitäts-Diskriminatorstufe zur Ausgabe eines ersten Stabilitätssignals, falls die Stabilität der Herzrate oberhalb eines vorbestimmten Schwellwertes liegt, und eines zweiten Stabilitätssignals, falls die Stabilität der Herzrate unterhalb des vorbestimmten Schwellwertes liegt,
aufweist und die Auswertungs- und Steuereinrichtung zur Ausgabe des zweiten Therapiesteuersignals im Ansprechen auf die Erzeugung des ersten Stabilitätssignals und zur Ausgabe des dritten Therapiesteuersignals im Ansprechen auf die Erzeugung des zweiten Stabilitätssignals bei Vorliegen einer Herzrate innerhalb des zweiten Herzraten-Wertebereiches ausgebildet ist.

3. Herztherapiegerät nach einem der vorangehenden Ansprüche,
**dadurch gekennzeichnet, daß**
die Therapieeinrichtung einen Therapiespeicher mit mindestens drei frei adressierbaren Speicherbereichen zur Speicherung des ersten bis dritten Therapiesteuersignals aufweist, wobei der erste und dritte Speicherbereich direkt durch die Herzraten-Diskriminatorstufe adressierbar sind, während der zweite Speicherbereich ausschließlich und der dritte Speicherbereich zusätzlich über UND-Glieder adressierbar sind, welche eingangsseitig mit den Ausgängen der Herzraten-Diskriminatorstufe und der Stabilitäts-Auswertungseinrichtung verbunden sind.

4. Herztherapiegerät nach einem der vorangehenden Ansprüche,
**gekennzeichnet durch**
die Ausbildung als kombinierter automatischer Herzschrittmacher/Defibrillator mit
- einer Schrittmachereinrichtung, die mindestens im Ansprechen auf das zweite Therapiesteuersignal, wahlweise auch im Ansprechen auf das erste Therapiesteuersignal, eine vorbestimmte Stimulationsimpulsfolge an das Herz des Patienten liefert und
- einem Defibrillator, der im Ansprechen an das dritte Therapiesteuersignal einen Schockimpuls an das Herz des Patienten liefert.

5. Herztherapiegerät nach Anspruch 4,
**dadurch gekennzeichnet, daß**
die Schrittmacherstufe zur Ausgabe verschiedener Stimulationsimpulsfolgen als Schrittmachertherapien abgestufter Aggressivität im Ansprechen auf das erste und zweite Therapiesteuersignal ausgebildet ist, wobei im Ansprechen auf das erste Therapiesteuersignal, welches eine Herzrate im ersten Wertebereich kennzeichnet, eine weniger aggressive Schrittmachertherapie ausgegeben wird als im Ansprechen auf das zweite Therapiesteuersignal, welches eine im zweiten Wertebereich liegende Herzrate mit hoher Stabilität kennzeichnet.

6. Herztherapiegerät nach Anspruch 1 und 4 oder 5,
**dadurch gekennzeichnet, daß**
die Schrittmacherstufe im Ansprechen auf das zweite Therapiesteuersignal, welches eine im zweiten Wertebereich liegende atriale Herzrate mit hoher Stabilität kennzeichnet, eine Hochfrequenz-Burst- oder eine Ramp-Impulsfolge ausgibt.

7. Herztherapiegerät nach einem der vorangehenden Ansprüche,
**gekennzeichnet durch**
die Ausbildung als automatischer Defibrillator oder kombinierter automatischer Herzuschrittmacher/Defibrillator, wobei
der Defibrillator zur Ausgabe von Schockimpulsen mit unterschiedlichen einstellbaren Energien im Ansprechen auf das zweite oder dritte Therapiesteuersignal ausgebildet ist, wobei im Ansprechen auf das zweite Therapiesteuersignal, welches eine im zweiten Wertebereich liegende Herzrate mit hoher Stabilität kennzeichnet, ein Schockimpuls mit niedriger Energie und im Ansprechen auf das dritte Therapiesteuersignal, welches eine im dritten Wertebereich liegende Herzrate oder eine im zweiten Wertebereich liegende Herzrate mit niedriger Stabilität kennzeichnet, ein Schockimpuls mit hoher Energie ausgegeben wird.

8. Herztherapiegerät nach einem der Ansprüche 3 bis 7,
**gekennzeichnet durch**
eine Ablaufsteuerung, welche einen Zeitgeber aufweist der derart ausgebildet ist, daß sie unter Steuerung **durch** den Zeitgeber periodisch den aktuellen Wert der Herzrate von der Herzraten-Erfassungseinrichtung abfragt und eine Diskriminierung mit den gespeicherten Wertbereichsgrenzen auslöst, in deren Ergebnis ein Zugriffssteuer- und Adresssignal ausgegeben wird, das ein Auslesen einer Therapiesteuergröße aus dem Therapiespeicher bewirkt oder unterbindet.

## Claims

1. A cardiac treatment device having
- a heart rate detection unit for detecting the heart rate of a patient,
- an analysis and control unit, which is connected at least indirectly to the output of the heart rate detection unit, for analyzing a measured heart rate, including its assignment to one of at least two predetermined value ranges, and for outputting a treatment control signal as a function of this assignment, and
- a treatment unit, which is connected to the output of the analysis and control unit, for implementing at least one treatment using a predetermined treatment variable in response to the treatment control variable, i.e., as a function of the assignment of the measured heart rate to one of the value ranges,
the analysis and control unit
- having a three-range memory for storing a first, second, and third value range of comparative heart rates, which adjoin one another and have rising values of the heart rate,
- a heart rate discriminator, which is connected to the heart rate detection unit and the three-range memory, for assigning a measured heart rate to the first, second, or third value range and for outputting a corresponding first, second, or third discriminator output signal,
- a stability analysis unit for analyzing the heart rate stability over a predetermined period of time in response to the output of the second heart rate discriminator output signal, and
- a logical processing unit for processing the second discriminator output signal and the output signal of the stability detection unit to produce a second or third treatment control signal, while the first and third discriminator output signals are output directly as first and third treatment control signals,
**characterized in that** the heart rate detection unit is implemented to be connected to the atrium of the heart of the patient, in particular via at least one detection electrode and a measured signal line connected thereto, for detecting the atrial heart rate,
and the treatment unit is implemented for outputting at least two different, uniquely predetermined treatments in response to the second and third treatment control signals.

2. The cardiac treatment device according to claim 1,
**characterized in that** the stability analysis unit
- has a heart rate measured value memory for storing a predetermined number of heart rate measured values or all heart rate measured values in a predetermined period of time,
- a statistical processing unit, which is connected to the heart rate measured value memory, for statistical processing and determination of a stability value of the heart rate, and
- a stability discriminator stage for outputting a first ability signal if the stability of the heart rate is above a predetermined threshold value, and a second stability signal if the stability of the heart rate is below the predetermined threshold value,
and the analysis and control unit is implemented for outputting the second treatment control signal in response to the generation of the first ability signal and for outputting the third treatment control signal in response to the generation of the second stability signal if a heart rate within the second heart rate value range is provided.

3. The cardiac treatment device according to one of the preceding claims,
**characterized in that** the treatment unit has a treatment memory having at least three freely addressable memory areas for storing the first through third treatment control signals, the first and third memory areas being addressable directly by the heart rate discriminator stage, while the second memory area is exclusively addressable and the third memory area is additionally addressable via AND elements, which are connected at their inputs to the outputs of the heart rate discriminator stage and the stability analysis unit.

4. The cardiac treatment device according to one of the preceding claims,
**characterized by** its implementation as a combined automatic cardiac pacemaker/defibrillator having
- a pacemaker unit, which provides a predetermined stimulation pulse sequence to the heart of the patient in response to the second treatment control signal, alternately also in response to the first treatment control signal, and
- a defibrillator, which provides a shock pulse to the heart of the patient in response to the third treatment control signal.

5. The cardiac treatment device according to claim 4,
**characterized in that** the pacemaker stage is implemented for outputting different stimulation pulse sequences as pacemaker treatments of staged aggressiveness in response to the first and second treatment control signals, a less aggressive pacemaker treatment being output in response to the first treatment control signal, which identifies a heart rate in the first value range, and in response to the second treatment control signal, which identifies a heart rate lying in the second value range, having higher stability.

6. The cardiac treatment device according to claim 1 and 4 or 5,
**characterized in that** the pacemaker stage outputs a high-frequency burst or ramp pulse sequence in response to the second treatment control signal, which identifies an atrial heart rate, lying in the second value range, having higher stability.

7. The cardiac treatment device according to one of the preceding claims,
**characterized by** its implementation as an automatic defibrillator or combined automatic cardiac pacemaker/defibrillator,
the defibrillator being implemented for outputting shock pulses having differently settable energies in response to the second or third treatment control signal, a shock pulse having lower energy being output in response to the second treatment control signal, which identifies a heart rate, lying in the second value range, having higher stability, and a shock pulse having higher energy being output in response to the third treatment control signal, which identifies a heart rate lying in the third value range or a heart rate, lying in the second value range, having lower stability.

8. The cardiac treatment device according to one of claims 3 through 7,
**characterized by** a sequence controller which has a timer implemented in such a way that it periodically queries the current value of the heart rate from the heart rate detection unit under the control of the timer and triggers a discrimination using the stored value range limits, as a result of which an access control and addressing signal is output, which causes or suppresses a readout of a treatment control variable from the treatment memory.

## Revendications

1. Appareil de thérapie cardiaque comprenant
- un dispositif d'enregistrement de rythme cardiaque pour l'enregistrement du rythme cardiaque d'un patient,
- un dispositif d'analyse et de commande relié au moins indirectement à la sortie du dispositif d'enregistrement de rythme cardiaque pour l'analyse d'un rythme cardiaque mesuré, y compris son attribution à une d'au moins deux plages de valeurs prédéfinies, et pour la sortie d'un signal de commande de thérapie en fonction de cette attribution et
- un dispositif de thérapie relié à la sortie du dispositif d'analyse et de commande pour la réalisation d'au moins une thérapie avec une grandeur de commande de thérapie prédéfinie en réaction à la grandeur de commande de thérapie, donc en fonction de l'appartenance du rythme cardiaque mesuré à l'une des plages de valeurs,
le dispositif d'analyse et de commande présentant
- une mémoire de trois plages pour la mémorisation d'une première, d'une seconde et d'une troisième plages de valeur contiguës avec des valeurs croissantes du rythme cardiaque, de rythmes cardiaques de référence,
- un discriminateur de rythme cardiaque relié au dispositif d'enregistrement de rythme cardiaque et à la mémoire de trois plages pour l'attribution d'un rythme cardiaque mesuré à la première, à la seconde et à la troisième plages de valeur et pour la sortie, d'un second et d'un troisième signaux appropriés de sortie du discriminateur,
- un dispositif d'analyse de stabilité pour l'analyse de la stabilité de rythme cardiaque sur une période prédéfinie en réaction à l'édition du second signal de sortie du discriminateur de rythme cardiaque et
- une unité de traitement logique pour le traitement du second signal de sortie du discriminateur et d'un signal de sortie du dispositif de stabilité pour générer un second ou un troisième signaux de commande de thérapie, alors que le premier et le troisième signaux de sortie du discriminateur sont sortis directement en tant que premier et troisième signaux de commande de thérapie, **caractérisé en ce que**
le dispositif d'enregistrement de rythme cardiaque est réalisé en particulier pour être relié au moyen d'au moins une électrode d'enregistrement et d'une ligne de signal de mesure reliée à cette électrode, pour l'enregistrement du rythme cardiaque atrial avec l'oreillette du coeur du patient,
et **en ce que** le dispositif de thérapie est réalisé pour l'édition d'au moins deux thérapies différentes, clairement prédéfinies, en réaction au second et au troisième signaux de commande de thérapie.

2. Appareil de thérapie cardiaque selon la revendication 1, **caractérisé en ce que** le dispositif d'analyse de stabilité présente
- une mémoire de valeur de mesure de rythmes cardiaques pour le stockage d'un nombre prédéfini de valeurs de mesure de rythme cardiaque ou de toutes les valeurs de mesure de rythme cardiaque dans une période prédéfinie,
- une unité de traitement statistique reliée à la mémoire de valeur de mesure de rythme cardiaque pour le traitement statistique et la détermination d'une valeur de stabilité du rythme cardiaque et
- un niveau discriminateur de stabilité pour l'édition d'un premier signal de stabilité, lorsque la stabilité du rythme cardiaque se situe au-dessus d'une valeur seuil prédéfinie, et d'un second signal de stabilité, lorsque la stabilité du rythme cardiaque est disposée au-dessous de la valeur seuil prédéfinie,
et le dispositif d'analyse et de commande est réalisé pour l'émission du second signal de commande de thérapie en réaction à la génération du premier signal de stabilité et pour l'édition du troisième signal de commande de thérapie en réaction à la génération du deuxième signal de stabilité en cas de présence d'un rythme cardiaque à l'intérieur de la seconde plage de valeur de rythme cardiaque.

3. Appareil de thérapie cardiaque selon l'une quelconque des revendications précédentes,
**caractérisé en ce que** le dispositif de thérapie présente une mémoire de thérapie avec au moins trois zones de mémoire librement adressables pour le stockage du premier signal de commande de thérapie jusqu'au troisième signal de commande de thérapie, la première et la troisième zones de mémoire étant adressables directement par le niveau discriminateur de rythme cardiaque, tandis que la seconde zone de mémoire exclusivement et la troisième zone de mémoire en supplément sont adressables par des portes ET qui sont reliées côté entrée aux sorties du niveau discriminateur de rythme cardiaque et du dispositif d'analyse de stabilité.

4. Appareil de thérapie cardiaque selon l'une quelconque des revendications précédentes, **caractérisé par** la réalisation sous la forme d'un pacemaker, défibrillateur automatique mixte comprenant
- un dispositif de pacemaker, qui fournit au moins en réaction au second signal de commande de thérapie, en option également en réaction au premier signal de commande de thérapie, une séquence d'impulsions de stimulation prédéfinie au coeur du patient et
- un défibrillateur qui fournit en réaction au troisième signal de commande de thérapie une impulsion de choc au coeur du patient.

5. Appareil de thérapie cardiaque selon la revendication 4, **caractérisé en ce que** le niveau de pacemaker est réalisé pour la sortie de différentes séquences d'impulsions de stimulation sous la forme de thérapies de pacemaker d'agressivité échelonnée en réaction au premier et au second signaux de commande de thérapie, une thérapie de pacemaker moins agressive étant sortie en réaction au premier signal de commande de thérapie, qui caractérise une fréquence cardiaque dans la première plage de valeurs, que dans la réaction au second signal de commande de thérapie, qui caractérise un rythme cardiaque se situant dans la seconde plage de valeurs avec une stabilité élevée.

6. Appareil de thérapie cardiaque selon les revendications 1 et 4 ou 5, **caractérisé en ce que** le niveau de pacemaker émet en réaction au second signal de commande de thérapie, qui caractérise un rythme cardiaque atrial situé dans la seconde plage de valeurs avec une stabilité élevée, une séquence d'impulsions en rafale à haute fréquence ou une séquence d'impulsions Ramp.

7. Appareil de thérapie cardiaque selon l'une quelconque des revendications précédentes, **caractérisé par** la réalisation sous la forme d'un défibrillateur automatique ou d'un pacemaker/défibrillateur automatique mixte, le défibrillateur étant conçu pour sortir des impulsions de choc avec différentes énergies réglables en réaction au second ou au troisième signal de commande de thérapie, une impulsion de choc avec une faible énergie étant sortie en réaction au second signal de commande de thérapie, qui caractérise une stabilité cardiaque située dans la seconde plage de valeur avec une grande stabilité, et une impulsion de choc avec une grande énergie étant sortie en réaction au troisième signal de commande de thérapie, qui caractérise une fréquence cardiaque située dans la troisième plage de valeurs ou un rythme cardiaque situé dans la seconde plage de valeurs avec une faible stabilité.

8. Appareil de thérapie cardiaque selon l'une quelconque des revendications 3 à 7,
**caractérisé par** une commande séquentielle, qui présente une minuterie qui est conçue de telle sorte qu'elle interroge sous commande par la minuterie de façon périodique la valeur actuelle du rythme cardiaque par le dispositif d'enregistrement du rythme cardiaque et déclenche une discrimination avec les limites de plage de valeurs qui sont mémorisées, en conclusion desquelles est sorti un signal de commande d'accès et d'adresse, qui entraîne ou interdit une lecture d'une grandeur de commande de thérapie dans la mémoire de thérapie.
